# FASCICULE DE BREVET EUROPEEN

(11) **EP 0 931 272 B1**
(45) Date de publication et mention de la délivrance du brevet: **03.04.2002**
(21) Numéro de dépôt: 97943927.0
(22) Date de dépôt: 03.10.1997
(51) Int. Cl.: G01T 1/29, G03B 42/04

(54) **SYSTEME DE LOCALISATION DE CASSETTES DE PRISE D'IMAGES NUMERIQUES**
LOKALISIERUNGSSYSTEM VON KASSETTEN MIT DIGITALEN BILDDATEN
SYSTEM FOR LOCALISING DIGITAL IMAGING CASSETTES

(30) Priorité: 10.10.1996 FR 9612381
(43) Date de publication de la demande: 28.07.1999
(73) Titulaire: GE MEDICAL SYSTEMS SA, 78533 Buc Cedex (FR)
(72) Inventeur: HEIDSIECK, Robert, F-78150 Rocquencourt (FR); RIT, Vincent, F-59500 Douai (FR); PICARD, Catherine, F-92100 Boulogne (FR); BAUDET, Jean, Louis, F-75003 Paris (FR)
(74) Mandataire: Goode, Ian Roy
(86) Numéro de dépôt international: FR9701745
(87) Numéro de publication internationale: WO9815848

(56) Documents cités:
- EP-A- 0 603 709
- EP-A- 0 714 038
- DE-A- 3 609 527
- US-A- 4 070 582

## Description

La présente invention a pour objet un système de localisation de cassettes de prise d'images numériques pour un appareil de radiographie. De tels appareils de radiographie comprennent, en général, un émetteur de rayons X, un dispositif de support de la cassette pour la prise d'images, l'organe à radiographier étant disposé entre l'émetteur de rayons X et le dispositif de support de la cassette, un moyen pour traiter les informations ainsi obtenues et un rangement de la cassette lorsque celle-ci n'est pas en service.

On connaît, par exemple, des appareils de mammographie qui comprennent une source de rayons X disposés d'un côté de l'organe à radiographier, une table de support transparente aux rayons X, disposée de l'autre côté de l'organe à radiographier, un plateau de maintien réglable venant appliquer l'organe sur la table de support et un logement pour recevoir une cassette de prise d'images contenant un film impressionnable. Le logement est disposé dans la table de support.

Après qu'une image de l'organe a été prise, la cassette contenant le film impressionné est extraite de son logement et le film est développé.

De tels appareils servent en général à la recherche d'éventuels symptômes de cancer du sein. Une première opération consiste à effectuer un dépistage systématique qui ne nécessite que la prise d'un ou deux clichés. Si ces clichés révèlent des symptômes de cancer, on procède alors à une diagnostic plus approfondi qui nécessite un plus grand nombre de clichés, par exemple d'une zone particulière de l'organe, en vue de jouer sur le mode de représentation et de visualisation des images. Si le diagnostic révèle la présence d'un cancer, il peut être nécessaire d'effectuer une biopsie. On dispose alors un système de ponction sur l'appareil de radiologie. Le système de ponction comprend en général une aiguille pour effectuer le prélèvement dans une zone suspectée d'être cancéreuse, aux fins d'analyse et un porte-aiguille. L'appareil de radiologie sert alors à assurer le positionnement de l'aiguille. Le système de ponction peut également servir à la pose d'un hameçon équipé d'un fil destiné au repérage par le chirurgien d'une zone cancéreuse lors d'une opération.

Lors de l'utilisation du système de ponction, on effectue en général un premier cliché de centrage de la zone à ponctionner, puis, grâce à un mécanisme de basculement de la source de rayons X, on effectue un cliché avec un angle de +15° et un second cliché avec un angle de -15° dans le but d'obtenir par stéréotaxie les coordonnées tridimensionnelles d'un point particulièrement intéressant puis on procède, après l'entrée de l'aiguille dans l'organe, à au moins deux clichés de contrôle pour vérifier que l'aiguille est en place dans la zone à ponctionner. Dans la pratique, on arrive à un total de l'ordre de huit clichés lors de l'utilisation du système de ponction. Le développement d'un film impressionnable dure de 3 à 5 minutes par cliché.

Pendant toute la durée de l'opération de biopsie et du développement des clichés, l'organe reste parfaitement immobile par rapport à l'appareil de radiologie et est maintenu en compression entre la table et le plateau de maintien. La patiente doit donc rester plus de 30 minutes dans une position immobile et relativement pénible.

Dans le but de réduire le temps d'immobilisation de l'organe, les cassettes contenant des films impressionnables peuvent être remplacées par des cassettes contenant un moyen de prise d'images numériques capable de réaliser une prise d'images extrêmement rapide. Les opérations de biopsie sont ainsi beaucoup plus brèves et réduisent l'inconfort de ces examens. De plus, les cassettes de prise d'images numériques permettent une amélioration de la qualité du diagnostic. Pour des raisons économiques, il est souhaitable d'utiliser des cassettes de prise d'images numériques sans changer le reste de l'appareil de radiologie. La cassette doit être amovible de façon à pouvoir être disposée, soit sous la table lors d'un diagnostic, soit dans le système de ponction lors d'une biopsie.

Une cassette de prise d'images numériques comprend une enveloppe à l'intérieur de laquelle est disposé un dispositif de détection du signal radiographique. Ce dispositif de détection peut, par exemple, comprendre un scintillateur capable de transformer les rayons X incidents, en rayonnement, lumineux, une fibre optique permettant de filtrer la majeure partie du rayonnement X ayant traversé le scintillateur et protégeant les composants situés après ladite fibre optique, et une caméra matricielle à éléments de transfert de charge (CCD) formant une zone sensible. Pour assurer un positionnement satisfaisant de la cassette dans un logement, on munit en général la cassette d'un moyen de verrouillage. La cassette est également munie d'un câble électrique la reliant à un moyen de traitement d'informations, en général un micro-ordinateur.

Si la cassette est insérée dans un logement dans une position incorrecte ou sans verrouillage des moyens de verrouillage, la qualité des images risque de s'en trouver affectée. Il peut s'avérer nécessaire que le micro-ordinateur effectue, grâce à un logiciel adapté, des traitements d'images différents selon l'utilisation de la cassette, dans le système de ponction ou dans le logement de la table de support.

Les documents EP-A-0 603 709 et EP-A-0 714 038 décrivent l'utilisation de cassettes de prise d'images numériques pour un appareil de radiographie.

La présente invention a donc pour objet la réalisation d'un système de localisation de cassettes qui permette de vérifier le positionnement et le verrouillage satisfaisant de la cassette dans son logement et la localisation de la cassette dans le logement de la table de support, dans le système de ponction ou encore dans une position inactive.

Le système de localisation, selon l'invention, est destiné à des cassettes de prise d'images numériques dans un appareil de radiographie du type comprenant une source de rayons X, un moyen de traitement de l'information, un support de la cassette en position active et un rangement de la cassette en position inactive. La cassette comprend un moyen capteur de l'insertion de la cassette dans le support et un moyen capteur de l'identification de la cassette. On peut ainsi reconnaître le type de cassette utilisé ainsi que le lieu où se trouve la cassette.

Dans un mode de réalisation de l'invention, le moyen de traitement de l'information comprend un moyen de traitement des signaux numériques en provenance de la cassette et un moyen de commande de la source de rayons X.

Dans un mode de réalisation de l'invention, le système comprend un moyen pour empêcher l'introduction d'une cassette de format standard parallélépipèdique dans le rangement, le logement de la cassette dans le rangement étant pourvu de coins tronqués, et la cassette dont l'introduction est autorisée étant de format parallélépipèdique à coins tronqués. On évite ainsi l'introduction de cassettes de type classique dans le rangement d'un appareil de radiologie équipé du système de localisation.

Avantageusement, la cassette comprend un capteur d'insertion dans le support, sous la forme d'un relais mécanique.

Dans un mode de réalisation de l'invention, la cassette comprend un capteur d'identification dans le rangement, sous la forme d'un relais à déclenchement magnétique provoqué par un aimant solidaire du rangement.

Dans un mode de réalisation de l'invention, la cassette comprend un verrou de blocage dans le support, un capteur de verrouillage, sous la forme d'un contact électrique avec le support et un moyen de numérisation du signal du capteur de verrouillage pour transmettre l'information de verrouillage au moyen de traitement de l'information.

Dans un mode de réalisation de l'invention, le système comprend en outre, un dispositif de ponction capable d'effectuer une biopsie et comprenant un deuxième support de la cassette en position active. Le deuxième support peut comprendre un moyen pour empêcher l'introduction de cassettes de format standard parallélépipèdique, le logement de la cassette dans le deuxième support étant pourvu de coins tronqués, et les cassettes dont l'introduction est autorisée étant de format parallélépipèdique à coins tronqués. La cassette peut comprendre un capteur d'insertion et une capteur d'identification dans le deuxième support, sous la forme d'un relais mécanique et d'un relais à déclenchement magnétique provoqué par un aimant solidaire du deuxième support.

Avantageusement la cassette comprend un verrou de blocage dans le deuxième support, un capteur de verrouillage, sous la forme d'un contact électrique avec le deuxième support, et un moyen de numérisation du signal de capteur de verrouillage pour transmettre une information de verrouillage au moyen de traitement de l'information.

Dans un mode de réalisation de l'invention, la cassette est équipée de câbles de transmission avec le moyen de traitement de l'information et comprend une poignée destinée à la préhension de la cassette et la protection de la sortie du câble de la cassette.

Grâce à l'invention, l'opérateur sait instantanément où se trouve la cassette qu'il recherche et évite, de plus, toute utilisation de la cassette lors d'un défaut de verrouillage ou d'un mauvais positionnement dans un support.

L'invention sera mieux comprise à l'étude de la description détaillée de modes de réalisation pris à titre d'exemples nullement limitatifs et illustrés par les dessins annexés sur lesquels :
la figure 1 est une vue d'ensemble en perspective du système de radiologie;
la figure 2 est une vue de face en élévation de la cassette de prise d'images;
la figure 3 est une vue de dessus en élévation de la cassette de prise d'images;
la figure 4 est une vue de côté en élévation de la cassette de prise d'images; et
les figures 5 et 6 sont des vues de dessus d'un autre mode de réalisation d'une cassette selon l'invention.

Tel qu'il est illustré sur les figures, le système de mammographie comprend un mammographe 1, un système de ponction 2, une cassette 3 de prise d'images numériques et un moyen de commande et de traitement 4. L'appareil de radiologie comprend une base 5 reposant sur le sol et supportant un plateau porte-sein 6 également appelé "bucky" de hauteur réglable et une source de rayons X 7 qui peut être basculée de ±30° par rapport au plan vertical de symétrie de l'appareil de radiologie 1. La source de rayons X 7 est supportée par une colonne 8 pourvue sur sa face avant d'une pluralité de trous 9 de fixation du système de ponction 2.

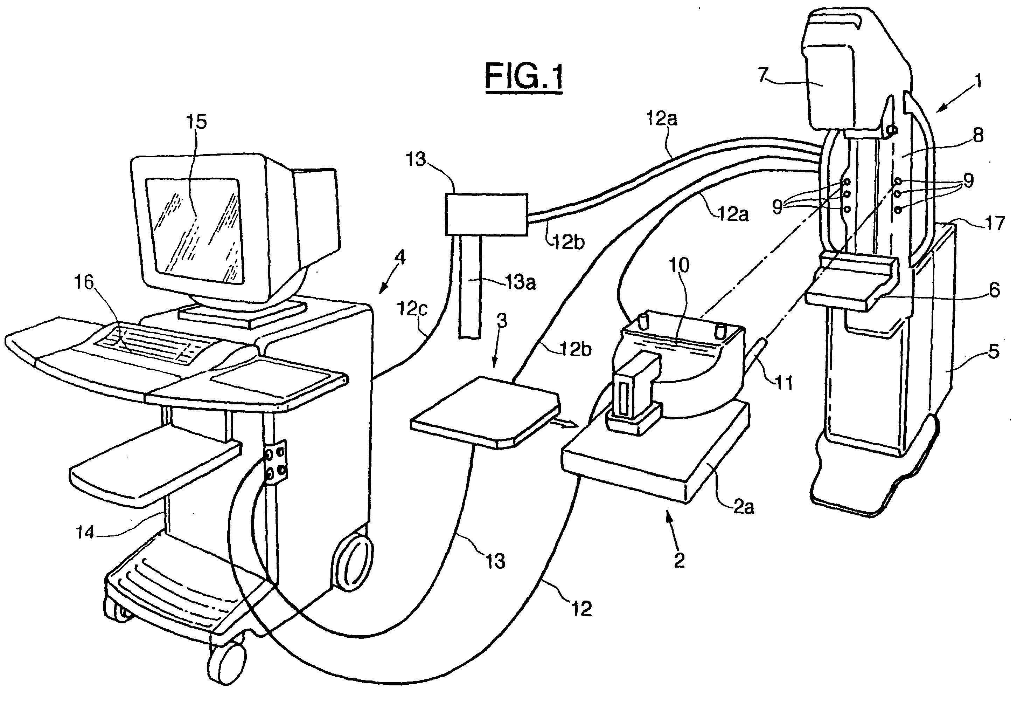

Le système de ponction 2 peut être monté ou non sur le mammographe 1 et comprend un plateau de maintien 2a jouant le rôle de pelote de compression, un porte-aiguille 10 et une aiguille non représentée capable d'effectuer une biopsie dans l'organe à radiographier. Le système de ponction 2 est équipé de deux broches 11, dont une seule est visible sur la figure 1, capables de venir se loger dans les trous 9 de la colonne 8 du mammographe 1, et de grenouillères, non représentées de fixation sur la colonne 8. Le système de ponction 2 est relié par un câble électrique 12a à une boîte de connexion 13.

La cassette 3 de prise d'images numériques est plate et sensiblement parallélépipèdique à coins tronqués, et est reliée par un câble électrique 12b à la boîte de connexion 13.

Celle-ci est reliée au moyen de commande et de traitement 4 et comprend un bouton de démarrage et d'arrêt, l'alimentation générale du système et les liaisons informatiques nécessaires, non représentés. La boîte de connexion 13 peut être montée sur une colonne 13a dans laquelle sont rangées les longueurs de câbles en surplus. Les câbles 12a et 12b passent par le mammographe 1 de façon à limiter le rayon de déplacement de la cassette 3 et du système de ponction 2. On réduit ainsi les risques de mauvais rangement de ces derniers.

Le moyen de commande et de traitement 4 comprend un châssis 14 et des moyens électroniques non représentés, du type micro-ordinateur, reliés au système de ponction 2 et à la cassette 3 par le câble électrique 12c, la boîte de connexion 13 et les câbles électriques 12a et 12b pour traiter les informations reçues de la cassette 3 et commander le système de ponction 2, en particulier, le déplacement du porte-aiguille 10 lors d'une biopsie. Le moyen de commande et de traitement 4 est capable de commander la source de rayons X 7 et comprend également un écran 15 de visualisation des images de l'organe radiographié et un clavier 16. Le moyen de commande et de traitement 4 peut être équipé d'un logiciel destiné au calcul des coordonnées tridimensionnelles de points de l'organe radiographié à partir de deux images prises selon des angles différents grâce au pivotement de la source 7 de rayons X. On peut alors obtenir une excellente visualisation, soit de zones particulières de l'organe radiographié lors d'un diagnostic, soit du positionnement de l'aiguille dans l'organe radiographié lors d'une biopsie, en utilisant des méthodes d'affichage optimisées.

La cassette 3 peut entrer en suivant le sens de la flèche de la figure 1 dans un logement du système de ponction 2, ou dans un logement prévu dans un porte-cassette, non représenté, utilisé lors des examens de diagnostic et prévu pour être fixé sur le plateau porte-sein 6, ou encore en position de repos, dans un rangement 17 du mammographe 1.

La cassette 3 comprend de façon non représentée, une zone sensible avec un paroi supérieure transparente aux rayons X, un scintillateur capable de transformer les rayons X en lumière visible, une couche de fibres optiques destinée au transfert de la lumière visible et une caméra matricielle composée d'une pluralité de cellules à transfert de charge (CCD).

En fonctionnement, les rayons X sont émis par la source 7, traversent un plateau de maintien non représenté, l'organe radiographié, le plateau porte-sein 6, la paroi supérieure de la zone sensible de la cassette 3 et passent dans le scintillateur lequel, à la réception de rayons X, émet de la lumière visible transférée à la caméra matricielle par la couche de fibres optiques. La caméra matricielle permet la transformation de l'information reçue sous forme de lumière visible en une information sous la forme d'un signal électrique numérique transmis par les câbles électriques 12b et 12c au moyen de commande et de traitement 4.

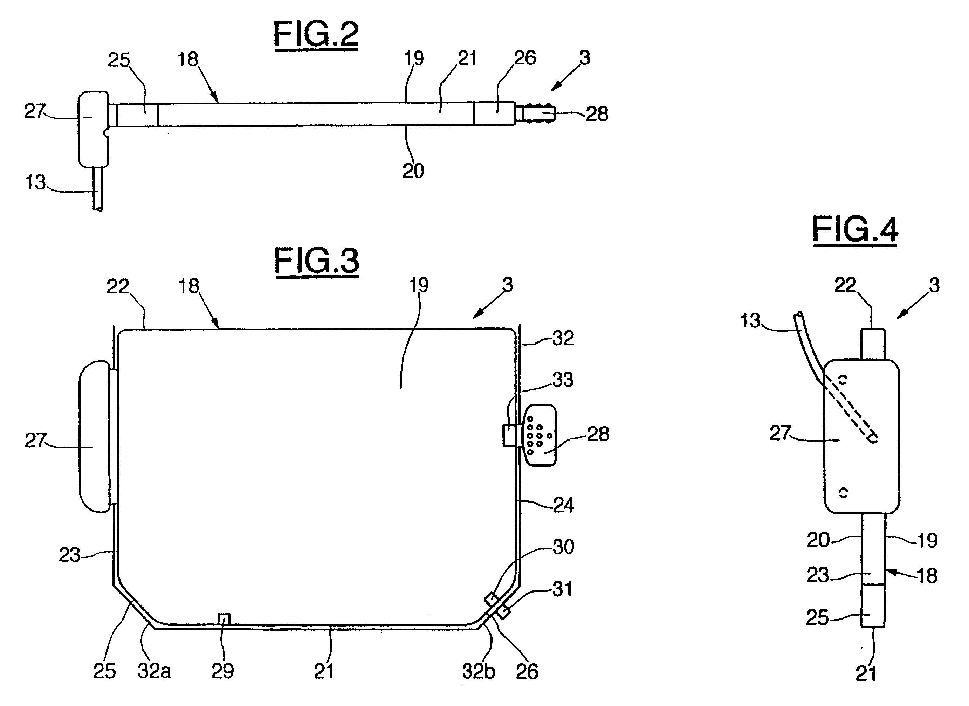

Comme on peut le voir sur les figures 2 à 4, la cassette 3 comprend un étui 18 sensiblement parallélépipèdique comprenant une face supérieure 19, une face inférieure 20, deux bords longitudinaux 21 et 22 et deux bords latéraux 23 et 24. Aux intersections entre le bord longitudinal 21 et le bord latéral 23 et entre le bord longitudinal 21 et le bord latéral 24 sont respectivement formés deux coins tronqués 25 et 26, tous deux perpendiculaires aux faces supérieure 19 et inférieure 20. Sur le bord latéral 23 de l'étui 18 de la cassette 3 est disposée une poignée 27 servant à la fois à la préhension de la cassette 3 par l'opérateur et à la protection du câble électrique 12b à sa sortie de l'étui 18 afin d'éviter qu'un mouvement du câble 12b par rapport à l'étui 18 n'entraîne une détérioration des contacts électriques de l'extrémité du câble 12b à l'intérieur de l'étui 18. La poignée 27 reste toutefois de faibles dimensions pour ne pas gêner la patiente pendant l'examen. Sur le bord latéral 24 opposé au bord latéral 23, la cassette 3 comprend un verrou 28 pour assurer le blocage de la cassette 3 dans son logement prévu soit dans le plateau porte-sein 6, le système de positionnement 2 ou le rangement 17 (figure 1).

La cassette 3 comprend sur son bord longitudinal 21 un capteur 29 de l'insertion de la cassette 3 dans le logement du système de positionnement 2 ou plateau porte-sein 6. Le capteur 29 est en général de type mécanique avec un poussoir. On pourrait ainsi prévoir un relais électromécanique qui serait déclenché par un déclencheur prévu dans les logements du système de positionnement 2 et du plateau porte-sein 6. La cassette 3 comprend également sur son coin tronqué 26 un capteur 30 d'identification de la cassette 3, en général sous la forme d'un relais à déclenchement magnétique capable d'être déclenché par un aimant 31 solidaire du logement du système de positionnement 2 et du rangement 17 dont un contour 32 est représenté schématiquement sur la figure 3.

Le contour 32 du logement est de forme adaptée pour recevoir la cassette 3 et comprend des coins tronqués 32a et 32b correspondant aux coins tronqués 25 et 26 de la cassette 3. Grâce aux coins tronqués 32a et 32b du contour 32 du logement, on empêche l'insertion complète d'une cassette de type classique dans un logement prévu pour une cassette 18 selon l'invention. Si un opérateur tente d'introduire une cassette de type classique dans un tel logement, les coins carrés de la cassette vont venir buter contre les coins tronqués 32a et 32b du contour 32 du logement et empêcheront l'insertion complète de celle-ci dans le logement. L'opérateur percevra immédiatement la cause du défaut d'insertion et sera conduit à utiliser la cassette du type souhaité. Les capteurs 29 et 30 de la cassette 3 permettent sa localisation instantanée en utilisant les informations qui sont envoyées à partir des capteurs 29 et 30 par le câble électrique 13 au moyen de commande et de traitement 4. On obtient la table de vérité suivante :

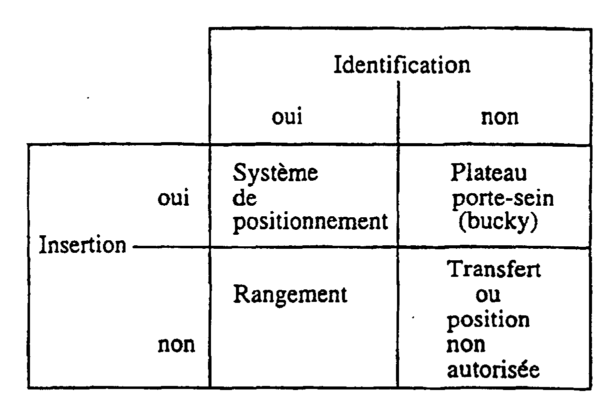

Lorsque le moyen de traitement et de commande 4 reçoit une information en provenance du capteur d'insertion 29 et une information en provenance du capteur d'identification 30, la cassette 3 est insérée dans le logement du système de positionnement 2. En effet, le système de positionnement 2 est capable de déclencher simultanément les capteurs d'insertion 29 et d'identification 30.

Lorsque le moyen de traitement et de commande 4 reçoit un signal du capteur d'insertion 29 et ne reçoit pas de signal du capteur d'identification 30, la cassette est insérée dans le plateau porte-sein 6. En effet, le plateau porte-sein 6 est capable de faire réagir le capteur mécanique d'insertion 29 mais est dépourvue d'aimant capable de faire réagir le capteur d'identification 30.

Lorsque le moyen de traitement et de commande 4 reçoit un signal du capteur d'identification 30 et ne reçoit pas un signal du capteur d'insertion 29, la cassette est insérée dans le logement du rangement 17 ce qui ne fait pas réagir le capteur d'insertion 29, ledit logement étant pourvu d'un aimant 31 capable de faire réagir le capteur d'identification 30.

Lorsque le moyen de traitement et de commande 4 ne reçoit aucun signal, ni du capteur d'insertion 29, ni du capteur d'identification 30, la cassette 3 n'est insérée dans aucun des logements qui lui sont destinés et peut être en cours de transfert de l'un à l'autre ou dans un logement non autorisé. L'opérateur est ainsi tenu informé en permanence du lieu où se trouve la cassette 3.

Le verrou 28 de la cassette 3 comprend un capteur de verrouillage 33 et un convertisseur analogique/numérique non représentée, qui envoie un signal numérique au moyen de traitement et de commande 4 lorsque le verrouillage du verrou 28 est effectué ce qui peut permettre, par exemple, d'empêcher la prise d'un cliché tant que la cassette 3 n'est pas verrouillée de façon satisfaisante dans le logement du système de positionnement 2 ou du plateau porte-sein 6. On évite ainsi une émission inutile de rayons X.

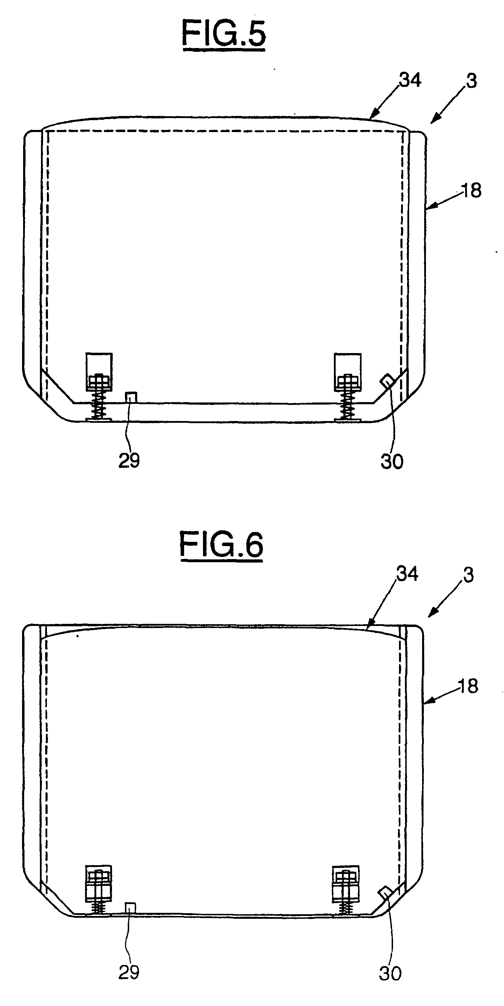

Les figures 5 et 6 illustrent un autre mode de réalisation de l'invention où une cassette 3 comprend un étui 18 dans lequel peut coulisser une boîte 34. Ce mouvement relatif entre la boîte 34 et l'étui 18 permet d'écarter la zone sensible de la cassette 3 située dans la boîte 34 du bord de l'étui 18 et de diminuer le risque de détérioration par choc. Les capteurs d'insertion 29 et de positionnement 30 peuvent être disposés de la même façon que sur les figures 2 à 4 ou pourraient également être disposés sur les bords latéraux de la cassette 3. Bien entendu, on peut utiliser pour les capteurs 29 et 30 tout type de capteur compact et capable d'envoyer un signal à un moyen de traitement et de commande, par exemple un contacteur électrique à la place d'un relais à déclenchement magnétique. On pourrait également utiliser une zone sensible formée d'une matrice de photodiodes à la place de la zone sensible formée d'une matrice d'éléments à transfert de charge.

Grâce à l'invention, on obtient une cassette de prise d'images numériques qui peut être localisée facilement et dont le rangement peut être reconnu rapidement par l'opérateur. On facilite ainsi l'utilisation de l'appareil de radiologie.

## Revendications

1. Système de localisation de cassettes (3) de prise d'images numériques dans un appareil de radiographie (1) du type comprenant une source de rayons X (7), un moyen de traitement de l'information (4), un support de la cassette en position active et un rangement (17) de la cassette (3) en position inactive, **caractérisé par le fait que** la cassette comprend un moyen capteur (29) de l'insertion de la cassette (3) dans ledit support et un moyen capteur (30) de l'identification de la cassette (3)

2. Système selon la revendication 1, **caractérisé par le fait que** le moyen (4) de traitement de l'information comprend un moyen de traitement des signaux numériques en provenance de la cassette (3) et un moyen de commande de la source de rayons X.

3. Système selon la revendication 1 ou 2, **caractérisé par le fait qu'**il comprend un moyen pour empêcher l'introduction d'une cassette de format standard parallélépipèdique dans le rangement, le logement de la cassette (3) dans ledit support et dans le rangement étant pourvu de coins tronqués (32a, 32b), et les cassettes (3) dont l'introduction est autorisée étant de format parallélépipèdique à coins tronqués (25, 26).

4. Système selon l'une quelconque des revendications précédentes, **caractérisé par le fait que** la cassette (3) comprend un capteur d'insertion (29) dans le support, sous la forme d'un relais mécanique.

5. Système selon l'une quelconque des revendications précédentes, **caractérisé par le fait que** la cassette (3) comprend un capteur d'identification (30) dans le rangement, sous la forme d'un relais à déclenchement magnétique provoqué par un aimant (31) solidaire du rangement.

6. Système selon l'une quelconque des revendications précédentes, **caractérisé par le fait que** la cassette (3) comprend un verrou (28) de blocage dans le support, un capteur de verrouillage (33), sous la forme d'un contact électrique avec le support et un moyen de numérisation du signal du capteur de verrouillage pour transmettre une information de verrouillage au moyen de traitement de l'information (4).

7. Système selon l'une quelconque des revendications précédentes, **caractérisé en ce qu'**il comprend, en outre, un dispositif de ponction (2) capable d'effectuer une biopsie et comprenant un deuxième support de la cassette (3) en position active.

8. Système selon la revendication 7, **caractérisé par le fait que** le deuxième support comprend un moyen pour empêcher l'introduction de cassettes de format standard parallélépipèdique, le logement de la cassette (3) dans le deuxième support étant pourvu de coins tronqués, et les cassettes (3) dont l'introduction est autorisée étant format parallélépipèdique à coins tronqués.

9. Système selon la revendication 7 ou 8, **caractérisé par le fait que** la cassette (3) comprend un capteur d'insertion et un capteur d'identification dans le deuxième support, sous la forme d'un relais mécanique et d'un relais à déclenchement magnétique provoqué par un aimant solidaire du deuxième support.

10. Système selon l'une quelconque des revendications 7 à 9, **caractérisé par le fait que** la cassette (3) comprend un verrou de blocage dans le deuxième support, un capteur de verrouillage, sous la forme d'un contact électrique avec le deuxième support, et un moyen de numérisation du signal du capteur de verrouillage pour transmettre une information de verrouillage au moyen de traitement de l'information.

11. Système selon l'une quelconque des revendications précédentes, **caractérisé par le fait que** la cassette (3) est équipée de câbles de transmission avec le moyen de traitement de l'information et comprend une poignée (27) destiné à la préhension de la cassette (3) et à la protection de la sortie du câble de la cassette (3).

## Patentansprüche

1. Vorrichtung zum örtlichen Festlegen von digitalen Bildaufnahmekassetten (3) in einem Durchstrahlungsgerät (1) eines Typs, der eine Röntgenstrahlungsquelle (7), eine Informationsverarbeitungseinrichtung (4), einen Halter zum Halten der Kassette in einer aktiven Position und eine Ablage (17) zum Ablegen der Kassette (3) in einer inaktiven Position umfaßt, **dadurch gekennzeichnet, daß** die Kassette eine Sensoreinrichtung (29) für das Einsetzen der Kassette (3) in den besagten Halter und eine Sensoreinrichtung (30) für die Identifikation der Kassette (3) umfaßt.

2. Vorrichtung nach Anspruch 1 **dadurch gekennzeichnet, daß** die Informationsverarbeitungseinrichtung (4) eine Einrichtung zum Verarbeiten der von der Kassette (3) stammenden digitalen Signale und eine Einrichtung zum Steuern der Röntgenstrahlungsquelle umfaßt.

3. Vorrichtung nach Anspruch 1 oder 2 **dadurch gekennzeichnet, daß** sie eine Einrichtung zum Verhindern des Einsetzens einer Kassette mit Standardparallelepipedformat in der Ablage umfaßt, die Aufnahme der Kassette (3) im besagten Halter und in der Ablage mit abgeschnittenen Ecken (32a, 32b) versehen ist und die Kassetten (3), deren Einsetzen erlaubt ist, ein Parallelepipedformat mit abgeschnittenen Ecken (25, 26) haben.

4. Vorrichtung nach einem der vorhergehenden Ansprüche **dadurch gekennzeichnet, daß** die Kassette (3) einen Sensor für das Einsetzen in den Halter in Form eines mechanischen Schalters umfaßt.

5. Vorrichtung nach einem der vorhergehenden Ansprüche **dadurch gekennzeichnet, daß** die Kassette (3) einen Sensor (30) zum Identifizieren in der Ablage in Form eines Relais umfaßt, das magnetisch durch einen Magneten (31) ausgelöst abfällt, der fest mit der Ablage verbunden ist.

6. Vorrichtung nach einem der vorhergehenden Ansprüche **dadurch gekennzeichnet, daß** die Kassette (3) einen Riegel (28) zum Sperren im Halter, einen Verriegelungssensor (33) in Form eines elektrischen Kontaktes mit dem Halter und eine Einrichtung zum Digitalisieren des Signals des Verriegelungssensors umfaßt, um eine Verriegelungsinformation auf die Informationsverarbeitungseinrichtung (4) zu übertragen.

7. Vorrichtung nach einem der vorhergehenden Ansprüche **dadurch gekennzeichnet, daß** sie weiterhin eine Punktionseinrichtung (2) umfaßt, die eine Biopsie ausführen kann und einen zweiten Halter zum Halten der Kassette (3) in einer aktiven Position umfaßt.

8. Vorrichtung nach Anspruch 7 **dadurch gekennzeichnet, daß** der zweite Halter eine Einrichtung zum Verhindern des Einsetzens von Kassetten mit Standardparallelepipedformat umfaßt, die Aufnahme der Kassette (3) im zweiten Halter mit abgeschnittenen Ecken versehen ist und die Kassetten (3), deren Einsetzen erlaubt ist, ein Parallelepipedformat mit abgeschnittenen Ecken haben.

9. Vorrichtung nach Anspruch 7 oder 8 **dadurch gekennzeichnet, daß** die Kassette (3) einen Sensor für das Einsetzen und einen Sensor für das Identifizieren im zweiten Halter in Form eines mechanischen Schalters und eines Relais umfaßt, das magnetisch über einen fest mit den zweiten Halter verbundenen Magneten ausgelöst abfällt.

10. Vorrichtung nach einem der Ansprüche 7 bis 9 **dadurch gekennzeichnet, daß** die Kassette (3) einen Riegel zum Sperren im zweiten Halter, einen Verriegelungssensor in Form eines elektrischen Kontaktes mit den zweiten Halter und eine Einrichtung zum Digitalisieren des Signals des Verriegelungssensors umfaßt, um eine Verriegelungsinformation auf die Informationsverarbeitungseinrichtung zu übertragen.

11. Vorrichtung nach einem der vorhergehenden Ansprüche **dadurch gekennzeichnet, daß** die Kassette (3) mit Leitungen zur Informationsübertragung auf die Informationsverarbeitungseinrichtung ausgerüstet ist und einen Griff (27) umfaßt, der dazu bestimmt ist, die Kassette (3) zu ergreifen und den Leitungsausgang der Kassette (3) zu schützen.

## Claims

1. System for locating cassettes (3) for producing digital images, in a radiography apparatus (1) of the type comprising an X-ray source (7), a data processing means (4), a support for the cassette in the active position and a storage space (17) for the cassette (3) in the inactive position, **characterized in that** the cassette comprises a means (29) for detecting the insertion of the cassette (3) into said support and a means (30) for detecting the identification of the cassette (3).

2. System according to Claim 1, **characterized in that** the data processing means (4) comprises a means for processing the digital signals output by the cassette (3), and a means for controlling the X-ray source.

3. System according to Claim 1 or 2, **characterized in that** it comprises a means for preventing the introduction of a cassette of standard parallelepipedal format into the storage space, the housing for the cassette (3) in said support and in the storage space being provided with truncated corners (32a, 32b), and the cassettes (3) whose introduction is allowed being of parallelepipedal format with truncated corners (25, 26).

4. System according to any one of the preceding claims, **characterized in that** the cassette (3) comprises a detector (29) for detecting insertion into the support, which is in the form of a mechanical relay.

5. System according to any one of the precedingclaims, **characterized in that** the cassette (3) comprises a detector (30) for identification in the storage space, which is in the form of a relay magnetically triggered by a magnet (31) fitted to the storage space.

6. System according to any one of the preceding claims, **characterized in that** the cassette (3) comprises a lock (28) for immobilization in the support, a locking detector (33) which is in the form of an electrical contact connecting to the support, and a means for digitizing the signal from the locking detector in order to send locking data to the data processing means (4).

7. System according to any one of the preceding claims, **characterized in that** it furthermore comprises a puncture device (2) capable of performing a biopsy and comprising a second support for the cassette (3) in the active position.

8. System according to Claim 7, **characterized in that** the second support comprises a means for preventing the introduction of cassettes of standard parallelepipedal format, the housing for the cassette (3) in the second support being provided with truncated corners, and the cassettes (3) whose introduction is allowed being of parallelepipedal format with truncated corners.

9. System according to Claim 7 or 8, **characterized in that** the cassette (3) comprises an insertion detector and an identification detector relating to the second support, which are in the form of a mechanical relay and a relay magnetically triggered by a magnet fitted to the second support.

10. System according to any one of Claims 7 to 9, **characterized in that** the cassette (3) comprises a lock for immobilization in the second support, a locking detector which is in the form of an electrical contact connecting to the second support, and a means for digitizing the signal from the locking detector in order to send locking data to the data processing means.

11. System according to any one of the preceding claims, **characterized in that** the cassette (3) is equipped with cables for transmission to the data processing means and comprises a handle (27) intended for the cassette (3) to be gripped and for the exit of the cable from the cassette (3) to be protected.
